# EUROPÄISCHE PATENTSCHRIFT

(11) **EP 1 326 855 B1**
(45) Veröffentlichungstag und Bekanntmachung des Hinweises auf die Patenterteilung: **23.01.2008**
(21) Anmeldenummer: 01982384.8
(22) Anmeldetag: 01.10.2001
(51) Int. Cl.: C07D 321/10, C09K 19/58, C07B 41/04

(54) **VERFAHREN ZUR HERSTELLUNG VON CYCLISCHEN CARBONSÄUREORTHOESTERFLUORIDEN UND SOLCHE VERBINDUNGEN**
METHOD FOR PRODUCING CYCLIC CARBOXYLIC ORTHOESTER FLUORIDES AND CORRESPONDING COMPOUNDS
PROCEDE DE PREPARATION DE FLUORURES D'ORTHOESTER D'ACIDE CARBOXYLIQUE CYCLIQUES ET COMPOSES CORRESPONDANTS

(30) Priorität: 20.10.2000 EP 00122844; 02.02.2001 DE 10105313
(43) Veröffentlichungstag der Anmeldung: 16.07.2003
(73) Patentinhaber: Merck Patent GmbH, 64293 Darmstadt (DE)
(72) Erfinder: KIRSCH, Peer, 64293 Darmstadt (DE); TAUGERBECK, Andreas, 64285 Darmstadt (DE)
(86) Internationale Anmeldenummer: PCT/EP2001/011323
(87) Internationale Veröffentlichungsnummer: WO 2002/034740

(56) Entgegenhaltungen:
- WO-A-01/64667
- WO-A-96/03357
- MUFFLER H.: "Cyclisierung unter Beteiligung von Fluridionen." JOURNAL OF FLUORINE CHEMISTRY., Bd. 21, Nr. 2, 1982, Seiten 107-132, XP001053334 ELSEVIER SEQUOIA. LAUSANNE., CH ISSN: 0022-1139
- SONDEJ S C ET AL: "Gem-Difluoro compounds: a convenient preparation from ketones and aldehydes by halogen fluoride treatment of 1,3-dithiolanes" JOURNAL OF ORGANIC CHEMISTRY, AMERICAN CHEMICAL SOCIETY. EASTON, US, Bd. 51, 1986, Seiten 3508-3513, XP002085716 ISSN: 0022-3263

## Beschreibung

Die Erfindung betrifft ein Verfahren zur Herstellung von cyclischen Carbonsäureorthoesterfluoriden. Ferner werden neue, nach dem erfindungsgemäßen Verfahren erhältliche cyclischen Carbonsäureorthoesterfluoride aufgezeigt.

Cyclische Carbonsäureorthoesterfluoride können vor allem Verwendung finden als strukturelle Bestandteile von Flüssigkristallen, Pharmazeutika, Pflanzenschutzmitteln oder als Vorstufen für solche Produkte oder für die Herstellung von Polymeren.

Die wenigen bisher bekannten Verfahren zur Herstellung von Carbonsäureorthoesterfluoriden basieren entweder auf dem säurekatalysierten Austausch von Alkoxy- oder Acyloxy-Funktionen in Orthoesterderivaten gegen Fluor (G. Simchen, "Orthocarbonsäure-Derivate" in Houben-Weyl: Methoden der Organischen Chemie, Georg Thieme Verlag, Stuttgart, 1985, S. 54-63) oder auf der Umsetzung von Diolen mit Perfluorolefinen (Bayliff et al., J. Chem. Soc., Perkin Trans. I, 1987, 763-767) . Ein allgemein anwendbares Verfahren zur Herstellung von cyclischen Carbonsäureorthoesterfluoriden ist dagegen nicht bekannt.

H. Muffler et al. (Journal of Fluorine Chemistry (1982), 21, 2, 107-132) offenbarten die Herstellung eines 2-Fluor-1,3-dioxans aus der entsprechenden 2-Chlorverbindung.

Die Druckschrift WO 96/03357 offenbart den Austausch von Thioestergruppen durch Fluorid unter Anwendung von elementarem Fluor. S. Sondej et al, (J. Am. Chem. Soc. (1986), 51, S. 3508-3513) beschreiben ein ebensolches Verfahren unter Anwendung von alternativen Fluorierungsmitteln. Die Druckschrift WO 01/64667 offenbart Bis(alkylthio)carbeniumsalze zur Verwendung in Fluorierungsreaktionen zusammen mit einem einwertigen Alkohol als Reaktionspartner.

Eine Aufgabe der Erfindung ist es daher, ein Verfahren zur Herstellung von cyclischen Carbonsäureorthoesterfluoriden zur Verfügung zu stellen, das von einfach zugänglichen Edukten ausgeht, eine Isolierung von Zwischenprodukten nicht erfordert und die Produkte in guten Ausbeuten liefert.

Ferner liegt der Erfindung die Aufgabe zugrunde, neue, nach dem erfindungsgemäßen Verfahren erhältliche Produkte zu beschreiben und vorteilhafte Verwendungen dieser aufzuzeigen.

Die Aufgabe wird mit einem Verfahren gemäß Anspruch 1 gelöst. Die Unteranspruche betreffen vorteilhafte Varianten dieses Verfahrens.

Gegenstand der Erfindung ist damit ein Verfahren der eingangs genannten Art, bei dem
a) mindestens ein Bis(alkylthio)carbeniumsalz der Formel II, wie unten definiert, in Gegenwart mindestens einer Base mit mindestens einer organischen, mindestens zwei Hydroxylgruppen aufweisenden Verbindung der Formel III, wie unten definiert, umgesetzt wird
b) und anschließend, vorzugsweise in situ, der erhaltene Thioorthoester mit einem Fluorierungsmittel und einem Oxidationsmittel zum cyclischen Carbonsäureorthoesterfluorid der Formel I, wie unten definiert, oxidativ fluorodesulfuriert wird.

Ein Vorteil des erfindungsgemäßen Verfahrens ist die leichte Zugänglichkeit der Bis(alkylthio)carbeniumsalze und der Hydroxyverbindungen, insbesondere der Diole, als Ausgangsverbindungen.

Ferner erfolgt die oxidative Fluorodesulfurierung unter sehr milden, leicht basischen Bedingungen und ist daher im Gegensatz zu den konventionellen Methoden mit einer Vielzahl von ungeschützten funktionellen Gruppen, z.B. einer Nitrilgruppe, kompatibel.

Des weiteren ist es von besonderem Vorteil des erfindungsgemäßen Verfahrens, dass die Umsetzung ausgehend vom Carbeniumsalz und der Hydroxyverbindung zum Produkt in einem Reaktionsgemisch, also ohne Isolierung und Aufreinigung der Zwischenprodukte erfolgen kann. Die hierbei erzielbaren Ausbeuten sind bei geringer Nebenproduktbildung hoch.

Ferner betrifft ein Gegenstand der Erfindung neue, nach dem erfindungsgemäßen Verfahren erhältliche cyclische Carbonsäureorthoesterfluoride. Diese Verbindungen können als strukturelle Bestandteile von Flüssigkristallen, Pharmazeutika, Agrochemikalien oder Polymeren Verwendung finden. Ein weiteres Anwendungsgebiet ist der Einsatz als chirale Komponente oder Dotierstoff in Flüssigkristaltmischungen oder Polymeren. Diese Orthoesterfluoride können auch in Gegenwart einer oder mehrerer geeigneter Lewis-oder Brönstedt-Säuren als 1,1-Dialkoxyslkylierungsmittel zur Herstellung von cyclischen Ketalen oder cyclischen Orthocarbonsäureestem verwendet werden.

Es wird ein Bis(alkylthio)carbeniumsalz der Formel II 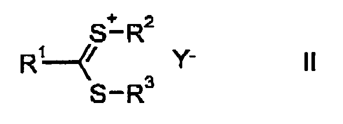 in dem erfindungsgemäßen Verfahren eingesetzt. Hierin bedeuten:
- R¹: geradkettiges, verzweigtes oder cyclisches Alkyl mit 1 bis 25 C-Atomen, worin ein oder mehrere H-Atome durch Halogen, -CN, weitere gegebenenfalls substituierte Alkyl- und/ oder Arylreste ersetzt sein können, und/ oder worin ein oder mehrere nicht benachbarte -CH₂-Gruppen unabhängig voneinander durch -CO-, -O-CO-, -CO-O-, -O-, -S-, -CH=CH-, -C≡C-, -NH- oder -N(CH₃)- ersetzt sein können, und/ oder
Aryl, das ein oder mehrfach mit Halogen, geradkettigem, verzweigtem und/ oder cyclischem Alkyl und/ oder Aryl substituiert sein kann, worin auch ein oder mehrere CH-Gruppen durch N oder O ersetzt sein können, bedeutet,
- R², R³: unabhängig voneinander geradkettiges, verzweigtes oder cyclisches Alkyl mit 1 bis 12 C-Atomen, wobei R², R³ derart
miteinander verbrückt sein können, dass die Gruppe 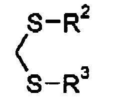 ein 4- bis 8-gliedriger Ring ist, und/ oder worin ein oder mehrere H-Atome durch Halogen, weitere gegebenenfalls substituierte Alkyl- und/ oder Arylreste ersetzt sein können, und/ oder worin ein oder mehrere nicht benachbarte -CH₂-Gruppen unabhängig voneinander durch -CO-, -O-, -S-, -CH=CH-, -C=C-, -NH- oder -N(CH₃)- ersetzt sein können, und/ oder
Aryl, das ein oder mehrfach mit Halogen, geradkettigem, verzweigtem und/ oder cyclischem Alkyl und/ oder Aryl substituiert sein kann, bedeuten und
- Y⁻: ein nicht- oder schwach-koordinierendes Anion ist.

Bevorzugt sind R² und R³ derart miteinander verbrückt, dass die Gruppe 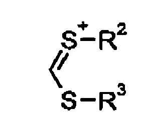 als 5- bis 7-gliedriger Ring 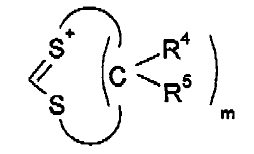 vorliegt, in der R⁴, R⁵ H oder eine gegebenenfalls substituierte Alkyl- oder Alkenylgruppe mit 1 bis 6 C-Atomen bedeutet, wobei die Gruppe 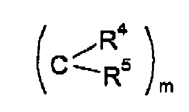 eine Cycloalkyl oder Arylgruppe ausbilden kann, und
m 2, 3 oder 4 ist.

Es wird eine organische, mindestens zwei Hydroxylgruppen aufweisende Verbindung der Formel III 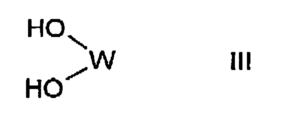 in dem erfindungsgemäßen Verfahren eingesetzt. Hierin bedeutet:
- W: eine Gruppe der Teilformel

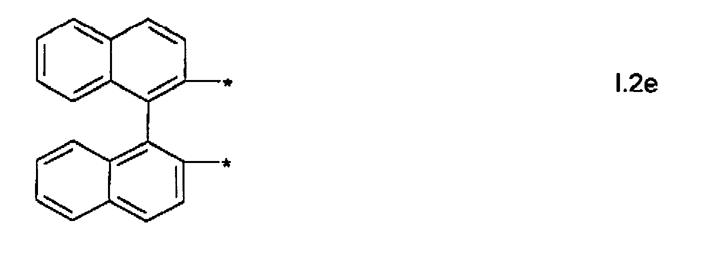

Die Umsetzung eines Bis(alkylthio)carbeniumsalzes der Formel II mit einer organischen, mindestens zwei Hydroxylgruppen aufweisenden Verbindung der Formel III ergibt erfindungsgemäß ein cyclisches Carbonsäureorthoesterfluorid der Formel I 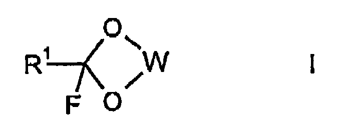 worin R¹ und W die angegebenen Bedeutungen besitzen.

Als organische, mindestens zwei Hydroxylgruppen aufweisende Verbindung wird 2,2'-Dihydroxy-1,1'-biphenyl eingesetzt.

In dem Bis(alkylthio)carbeniumsalz der Formel II ist Y⁻ bevorzugt ein Halogenid, Tetrafluoroborat, Hexafluorophosphat, Perchlorat oder Alkyl- oder Arylcarboxylat oder Alkyl- oder Arylsulfonat-Anion, wobei in den Alkyl- oder Arylgruppen ein, mehrere oder alle H-Atome durch Fluor oder Chlor substituiert sein können.

Als Oxidationsmittel können übliche Oxidationsmittel verwendet werden. Bevorzugt wird als Oxidationsmittel eine Verbindung eingesetzt, die Haloniumäquivalente freisetzt. Beispielhafte Oxidationsmittel sind N-Chlorsuccinimid, N-Bromsuccinimid, N-Jodsuccinimid, Dibromisocyanursäure, 1,3-Dibrom-5,5-dimethylhydanthoin, Chlor und Brom. Besonders bevorzugt ist Brom, da sich die entstehenden Bromide leicht abtrennen lassen. Ebenfalls geeignet sind auch SO₂Cl₂, SO₂ClF, Chloramin T, Nitrosonium- und Nitroniumsalze, beispielsweise NO⁺BF₄⁻. Die Nitrosonium- und Nitroniumsalze lassen sich gegebenenfalls auch in situ aus geeigneten Vorstufen, beispielsweise aus anorganischen oder organischen Nitriten und/ oder Nitraten, herstellen.

Als Fluorierungsmittel können übliche Fluorierungsmittel eingesetzt werden. Besonders bevorzugt wird das Fluorierungsmittel ausgewählt aus der Gruppe die gebildet ist von Fluorwasserstoff, aliphatischen und aromatischen Amin-Fluorwasserstoff-Komplexen, insbesondere ausgewählt ist aus der Gruppe, die gebildet ist von Pyridin-, Triethylamin-, Melamin-, Polyvinylpyridin-Fluorwasserstoff-Komplexen.

Bei der Durchführung des erfindungsgemäßen Verfahrens wird das Bis(alkylthio)carbeniumsalz zu der organischen, mindestens zwei Hydroxylgruppen aufweisenden Verbindung vorzugsweise in einem Molverhältnis von kleiner gleich 2 :1, insbesondere kleiner gleich 1,7 :1, besonders bevorzugt kleiner gleich 1,3 :1, ganz besonders bevorzugt kleiner gleich 1 : 1, eingesetzt. Der Einsatz des Carbeniumsalzes in etwa äquimolarer oder unterstöchiometrischer Menge zur Hydroxyverbindung führt im allgemeinen zur geringeren Bildung von Nebenprodukten. So können bei Molverhältnissen im Bereich größer gleich 2 : 1 durch Addition jeweils eines Bis(alkylthio)carbeniumions an eine Hydroxylgruppe über Dithioorthoester als Zwischenprodukte und anschließender Fluorodesulfurierung Verbindungen mit ein oder zwei CF₂O-Brücken entstehen.

Eine beispielhafte Durchführung des erfindungsgemäßen Verfahrens ist im folgenden angegeben. Eine Lösung des Bis(alkylthio)carbeniumsalzes wird mit einer Lösung der Hydroxyverbindung und mindestens einer Base versetzt, vorzugsweise in einem Temperaturbereich von -100 bis + 50°C. Hierzu vorteilhaft geeignete Basen sind organische Stickstoffbasen, insbesondere tertiäre aliphatische und/ oder aromatische Amine, wie beispielsweise Triethylamin, Pyridin oder Pyridin-Derivate. Die Base wird vorteilhaft in einem molaren Verhältnis 1 : 1 bis 2 : 1 bezogen auf die Hydroxyverbindung eingesetzt. Als geeignete Lösungsmittel kommen insbesondere polare Lösungsmittel oder Lösungsmittelgemische in Frage, beispielsweise Ether oder Halogenalkane, wie Diethylether, Tetrahydrofuran, Dichlormethan und/ oder Trichlormethan.

Durch Umsetzung der Bis(alkylthio)carbeniumsalze mit den Hydroxyverbindungen entstehende Zwischenprodukte, wie Thioorthoester, werden im allgemeinen nicht isoliert, sondern in dem Reaktionsgemisch direkt oxidativ zum cyclischen Carbonsäureorthoesterfluorid desulfuriert.

Hierzu wird das Reaktionsgemisch mit dem Fluorierungsmittel und dem Oxidationsmittel versetzt. Vorteilhaft wird hiernach die Temperatur des Gemisches erhöht, beispielsweise auf -20°C bis 50°C. Das cyclische Carbonsäureorthoesterfluorid kann nach dem Fachmann geläufigen Methoden, beispielsweise durch Umkristallisation und/ oder chromatographisch, aus dem Reaktionsgemisch erhalten werden.

Die Bis(alkylthio)carbeniumsalze als Edukte des erfindungsgemäßen Verfahrens sind nach bekannten Methoden leicht zugänglich, beispielsweise durch Kondensation von Carbonsäuren oder aktivierten Carbonsäurederivaten mit Dithiolen. Besonders vorteilhaft werden diese durch Addition einer Säure an ein Ketendithioketal erhalten. So ist das Bis(alkylthio)carbeniumsalz der Formel II durch Addition einer Säure HY, worin Y die zuvor angegebene Bedeutung hat, an ein Ketenditihioketal der Formel IV 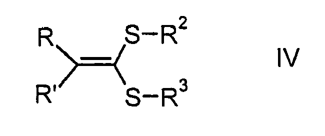 erhältlich. In der Formel IV besitzen R², R³ die zuvor genannten Bedeutungen und R, R' haben unabhängig voneinander derart eine der zuvor für R¹ angegebenen Bedeutungen, einschließlich H, dass die Gruppe 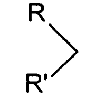 die selbe Bedeutung wie R¹ besitzt.

Die Säure wird in etwa äquimolarer Menge bezogen auf die umzusetzenden Ketendithioketal-Einheiten eingesetzt. Die Umsetzung mit der Säure HY erfolgt vorteilhaft in einem Temperaturbereich von -80 bis +30°C in einem inerten polaren Lösungsmittel oder Lösungsmittelgemisch, wie sie bereits zuvor beispielhaft genannt wurden. Hierbei besonders bevorzugte Säuren HY sind Trifluormethansulfonsäure und ein Tetrafluoroborsäure-Diethyletherkomplex.

Ein Vorteil dieser vom Ketendithioketal ausgehenden Verfahrensvariante ist die bereits unter sehr schonenden Bedingungen durchführbare Säureaddition an die Ketendithioketale. Hierdurch sind Bis(alkylthio)carbeniumsalze zugänglich, die empfindliche funktionelle Gruppen, wie Ester, Nitrile oder Ketale, enthalten.

Ferner besitzt diese Variante den Vorteil, dass die Bildung des Bis(alkylthio)carbeniumsalzes durch Addition der Säure an das Ketendithioketal reversibel ist. Bei Einsatz von in 4-Stellung substituierten Cyclohexylidenketendithioketalen lassen sich daher mit sehr hoher Selektivität die gegenüber der cis-Konfiguration thermodynamisch günstigeren trans-substituierten Cyclohexanderivate des Bis(alkylthio)carbeniumsalzes und damit trans-substituierte Cycloxanverbindungen mit einer Carbonsäureorthoesterfluorid-Funktion erhalten.

Zur Equilibrierung zum thermodynamisch günstigeren Isomer ist es daher vorteilhaft, das Reaktionsgemisch aus Ketendithioketal, Säure und entsprechendem Carbeniumsalz eine längere Zeit, insbesondere 15 Minuten bis 6 Stunden oder auch länger, bei einer Temperatur von -80 bis +50°C, insbesondere von -30 bis +50°C, zu rühren.

Die sich an die Umsetzung des Ketendithioketals anschließenden Syntheseschritte werden vorzugsweise in situ, also unter Verwendung des Reaktionsgemisches der ersten Umsetzung und damit ohne Isolierung des Carbeniumsalzes durchgeführt.

Gemäß einer weiteren Verfahrensvariante wird das Ketendithioketal aus einer Carbonylverbindung erhalten. Zum Erhalt eines Ketendithioketals der Formel IV wird eine Carbonylverbindung der Formel V 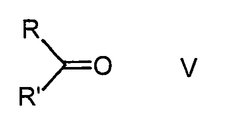 eingesetzt, in der R und R' die angegebenen Bedeutungen besitzen.

R und R' können unter Ausbildung einer cyclischen Gruppe miteinander verbunden sein. Beispiele hierfür sind Cyclohexanon und in 4-Stellung substituierte Cyclohexanon-Derivate.

Die Carbonylverbindung kann auch zwei oder mehr Carbonylfunktionen aufweisen. Beispiele hiefür sind Cyclohexan-1,4-dion und 2 Cyclohexanon-Gruppen aufweisende Verbindungen. Gemäß einer ersten Untervariante können alle Carbonylfunktionen zu Ketendithioketal-Funktionen und weiter zu Carbonsäureorthoesterfluorid-Funktionen erfindungsgemäß umgewandelt werden. Gemäß einer zweiten Untervariante wird vor der Umsetzung eine oder mehrere Carbonylfunktionen als Ketal geschützt, wobei mindestens eine Carbonylgruppe zur Umsetzung zum Ketendithioketal ungeschützt bleibt. Die hieraus erfindungsgemäß zugänglichen Carbonsäureorthoesterfluoride weisen neben der Orthoesterfluorid-Gruppe ein oder mehrere Carbonylfunktionen, gegebenenfalls geschützt als Ketal auf. Die freie Carbonyl-Funktion kann vorteilhaft zum Aufbau einer gegebenenfalls substituierten Alkyl-, Alkenyl- oder Alkoxygruppe verwendet werden.

Die Ketendithioketale sind aus den Carbonylverbindungen nach an sich bekannten Verfahren auf einfache Weise und in hohen Ausbeuten zugänglich. Beispielhaft sei hier D. J. Ager, Org. React. 1990, 38, 1-223, insbesondere die Seiten 63, 95 und 96 genannt. Von Vorteil ist es, dass die Carbonylverbindungen zusätzlich säurelabile Substituenten, beispielsweise Ketale oder Acetale zur Maskierung von Carbonylfunktionen, enthalten können. Ferner sind die hieraus erhältlichen Ketenthioketale in der Regel durch ihre guten Kristallisationseigenschaften gut zu reinigen, wobei dennoch eine gute Löslichkeit in üblichen organischen Lösungsmitteln gegeben ist.

Ein hierbei bevorzugtes Verfahren ist die Umsetzung einer Carbonylverbindung mit einem 2-Silyl-1,3-dithian, das substituiert sein kann. Besonders bevorzugt ist hierbei der Einsatz von 2-Trimethylsilyl-1,3-dithian. Die Umsetzung erfolgt vorzugsweise in Gegenwart einer deprotonierenden Verbindung, wie Alkyllithium, beispielsweise n-Butyllithium. Ein vorteilhafter Bereich der Reaktionstemperatur ist -130 bis 0°C. Geeignete Lösungsmittel sind die zuvor angegebenen Lösungsmittel bzw. Gemische.

Ausgehend von der Carbonylverbindung der Formel V über das Ketendithioketal der Formel IV und das durch Addition der Säure HY erhältliche Bis(alkylthio)carbeniumsalz der Formel II' ist durch Umsetzung mit der Hydroxyverbindung der Formel III und anschließender Fluorodesulfurierung das cyclische Carbonsäureorthoesterfluorid der Formel I' erfindungsgemäß herstellbar, wie in Reaktionsschema 1 angegeben.

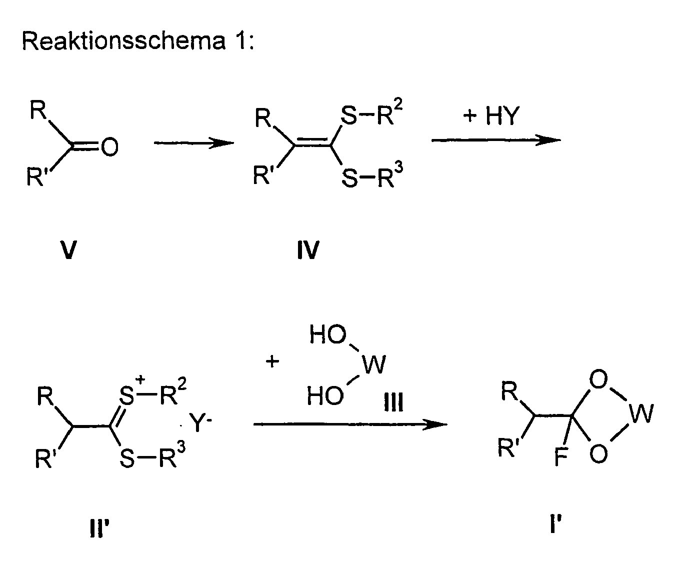

Die Formeln I' und II' sind für den Fall, dass die Gruppe 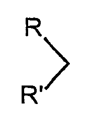 die selbe Bedeutung wie R¹ besitzt, mit den Formeln I bzw. II identisch.

Die gesamte Umsetzung von der Carbonylverbindung der Formel V bis zum cyclischen Carbonsäureorthoesterfluorid der Formel I' erfolgt besonders bevorzugt als sogenanntes Eintopfverfahren, d. h. ohne Isolierung und Aufreinigung von Zwischenprodukten.

Gegenstand der vorliegenden Erfindung sind ebenfalls die nach dem erfindungsgemäßen Verfahren erhältlichen cyclischen Carbonsäureorthoesterfluoride, insbesondere solche gemäß der Formel I 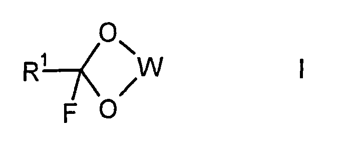 worin R¹ und W die zuvor angegebenen Bedeutungen besitzen.

Die Bedeutung der Formel I schließt alle Isotope der in den Verbindungen der Formel I gebundenen chemischen Elemente ein. Weisen Verbindungen der Formel I ein oder mehrere chirale Zentren auf, so umfasst die Formel I neben den Racematen auch enantiomerenreine und -angereicherte Formen. In enantiomerenreiner oder -angereicherter Form eignen sich die Verbindungen der Formel I auch als chirale Dotierstoffe und generell zur Erzielung chiraler Mesophasen.

Bevorzugt weist der Rest R¹ die Formel la auf 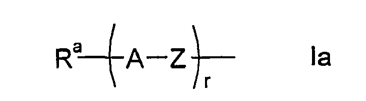 in der
- R^{a}: H, Halogen, -CN, -NCS, -SF₅ oder Alkyl mit 1 bis 12 C-Atomen, worin auch eine oder zwei nicht benachbarte -CH₂-Gruppen durch -O-, -S-, -CO-, -O-CO-, -CO-O-, -E- und/ oder -C=C- ersetzt sein können und/ oder worin auch eine oder mehrere H-Atome durch Halogen und/ oder -CN ersetzt sein können, bedeutet,
- E: CR⁴=CR⁵ oder CHR⁴-CHR⁵,
- R⁴, R⁵: jeweils unabhängig voneinander H, Alkyl mit 1-6 C-Atomen, F, Cl, Br, CF₃ oder CN bedeutet,
- Z: -O-CO-, -CO-O-, -C₂H₄-, -CH₂-CF₂-, -CF₂-CH₂-, -CF₂-CF₂-, -(CF₂)₃-, -(CF₂)₄-, -CH₂-O-, -CF₂-O-, -O-CH₂-, -O-CF₂-, -CH=CH-, -CF=CH-, -CH=CF-, -CF=CF-, -C≡C- oder eine Einfachbindung,
- A: 1,4-Phenylen, worin ein oder mehrere CH-Gruppen durch N ersetzt sein können, 1,4-Cyclohexylen, worin ein oder zwei nicht benachbarte CH₂-Gruppen durch O und/ oder S ersetzt sein können, 1,4-Cyclohexenylen, 1,4-Bicyclo(2,2,2)octylen, Piperidin-1,4-diyl, Naphthalin-2,6-diyl, Decahydronaphthalin-2,6-diyl oder 1,2,3,4-Tetrahydronaphthalin-2,6-diyl, wobei in diesen Gruppen ein oder mehrere H-Atome substituiert sein können durch Halogen, -CN und/ oder gegebenenfalls ein- oder mehrfach halogeniertes Alkyl mit 1 bis 6 C-Atomen, und
- r: 1, 2, 3 oder 4 ist, wobei mehrfach vorkommende Gruppen A und/oder Z gleiche oder verschiedene Bedeutungen besitzen können.

Vorzugsweise ist r 1, 2 oder 3.

Besonders bevorzugte Bedeutungen von A sind 1,4-Phenylen, das ein-, zwei- oder dreifach durch Fluor substituiert sein kann, trans-1,4-Cyclohexylen und 1,3-Dioxan-2,5-diyl, für die nachfolgend der Einfachheit halber die Abkürzungen Phe, Cyc bzw. Dio verwendet werden.

Der Begriff 1,3-Dioxan-2,5-diyl umfasst jeweils die beiden Stellungsisomeren

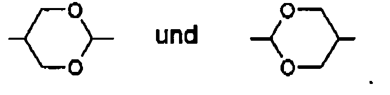

Folgende Reste R¹ der Teilformeln Ia.1 bis Ia.60 sind bevorzugt:

R^{a}- Ia.1

R^{a}-Cyc- Ia.2

R^{a}-Phe- Ia.3

R^{a}-Dio- Ia.4

R^{a}-Cyc-Cyc- Ia.5

R^{a}-Cyc-Phe- Ia.6

R^{a}-Cyc-Dio- Ia.7

R^{a}-Phe-Cyc- Ia.8

R^{a}-Phe-Phe- Ia.9

R^{a}-Phe-Dio- Ia.10

R^{a}-Dio-Cyc- Ia.11

R^{a}-Dio-Phe- Ia.12

R^{a}-Cyc-Z-Cyc- Ia.13

R^{a}-Cyc-Z-Phe- Ia.14

R^{a}-Cyc-Z-Dio- Ia.15

R^{a}-Phe-Z-Cyc- Ia.16

R^{a}-Phe-Z-Phe- Ia.17

R^{a}-Phe-Z-Dio- Ia.18

R^{a}-Dio-Z-Cyc- Ia.19

R^{a}-Dio-Z-Phe- Ia.20

R^{a}-Cyc-Cyc-Cyc- Ia.21

R^{a}-Cyc-Cyc-Phe- Ia.22

R^{a}-Cyc-Cyc-Dio- Ia.23

R^{a}-Cyc-Phe-Cyc- Ia.24

R^{a}-Cyc-Phe-Phe- Ia.25

R^{a}-Cyc-Phe-Dio- Ia.26

R^{a}-Cyc-Dio-Cyc- Ia.27

R^{a}-Cyc-Dio-Phe- Ia.28

R^{a}-Phe-Cyc-Cyc- Ia.29

R^{a}-Phe-Cyc-Phe- Ia.30

R^{a}-Phe-Cyc-Dio- Ia.31

R^{a}-Phe-Phe-Cyc- Ia.32

R^{a}-Phe-Phe-Phe- Ia.33

R^{a}-Phe-Phe-Dio- Ia.34

R^{a}-Phe-Dio-Cyc- Ia.35

R^{a}-Phe-Dio-Phe- Ia.36

R^{a}-Dio-Cyc-Cyc- Ia.37

R^{a}-Dio-Cyc-Phe- Ia.38

R^{a}-Dio-Phe-Cyc- Ia.39

R^{a}-Dio-Phe-Phe- Ia.40

R^{a}-Cyc-Z-Cyc-Z-Cyc- Ia.41

R^{a}-Cyc-Z-Cyc-Z-Phe- Ia.42

R^{a}-Cyc-Z-Cyc-Z-Dio- Ia.43

R^{a}-Cyc-Z-Phe-Z-Cyc- Ia.44

R^{a}-Cyc-Z-Phe-Z-Phe- Ia.45

R^{a}-Cyc-Z-Phe-Z-Dio- Ia.46

R^{a}-Cyc-Z-Dio-Z-Cyc- Ia.47

R^{a}-Cyc-Z-Dio-Z-Phe- Ia.48

R^{a}-Phe-Z-Cyc-Z-Cyc- Ia.49

R^{a}-Phe-Z-Cyc-Z-Phe- Ia.50

R^{a}-Phe-Z-Cyc-Z-Dio- Ia.51

R^{a}-Phe-Z-Phe-Z-Cyc- Ia. 52

R^{a}-Phe-Z-Phe-Z-Phe- Ia. 53

R^{a}-Phe-Z-Phe-Z-Dio- Ia. 54

R^{a}-Phe-Z-Dio-Z-Cyc- Ia. 55

R^{a}-phe-Z-Dio-Z-Phe- Ia. 56

R^{a}-Dio-Z-Cyc-Z-Cyc- Ia. 57

R^{a}-Dio-Z-Cyc-Z-Phe- Ia. 58

R^{a}-Dio-Z-Phe-Z-Cyc- Ia, 59

R^{a}-Dio-Z-Phe-Z-Phe- Ia. 60

Ganz besonders bevorzugte Bedeutungen von R^{a} sind F, Cl, CN oder Alkyl oder Alkoxy mit 1 bis 8 C-Atomen oder Alkenyl oder Alkenyloxy mit 2 bis 8 C-Atomen, wobei die Alkyl-, Alkoxy-, Alkenyl- oder Alkenyloxyreste auch ein- oder mehrfach halogeniert, insbesondere fluoriert sein können.

Bevorzugte Bedeutungen von Z sind -O-CO-, -CO-O-, -C₂H₄-, -CF₂-CF₂-, -CH₂-O-, -CF₂-O-, -O-CH₂-, -O-CF₂-, -CH=CH-, -C≡C- oder eine Einfachbindung.

Im Falle der Bedeutung Alkyl in den vorstehend oder nachfolgend angegebenen Gruppen oder Substituenten, insbesondere in R, R', R^{a}, R¹, R², R³, R⁴ und/ oder R⁵, kann der Alkyl-Rest linear oder verzweigt sein. Bevorzugt besitzt er 1, 2, 3, 4, 5, 6, 7 oder 8 C-Atome. Bevorzugt ist er linear und bedeutet daher besonders Methyl, Ethyl, Propyl, Butyl, Pentyl, Hexyl, Heptyl oder Octyl. Ein verzweigter Alkylrest kann chiral oder achiral sein. Bevorzugte chirale Alkylreste sind 2-Butyl (=1-Methylpropyl), 2-Methylbutyl, 2-Methylpentyl, 3-Methylpentyl, 2-Ethylhexyl, 2-Propylpentyl, 2-Octyl. Bevorzugte achirale Alkylreste sind Isopropyl, Isobutyl (=Methylpropyl), Isopentyl (=3-Methylbutyl). Die Alkylreste können in der angegebenen Weise substituiert sein.

Im Falle der Bedeutung Alkoxy in den vorstehend oder nachfolgend angegebenen Gruppen oder Substituenten, insbesondere in R^{a}, kann der Alkoxy-Rest linear oder verzweigt sein. Bevorzugt ist er linear und besitzt 1, 2, 3, 4, 5, 6, 7 oder 8 C-Atome und bedeutet daher besonders Methoxy, Ethoxy, Propoxy, Butoxy, Pentoxy, Hexoxy, Heptoxy oder Octoxy, des weiteren Nonoxy, Decoxy, Undecoxy, Dodecoxy, Tridecoxy oder Tetradecoxy.

Im Falle der Bedeutung Alkenyl in den vorstehend oder nachfolgend angegebenen Gruppen oder Substituenten, insbesondere in R^{a}, kann der Alkenylrest geradkettig oder verzweigt sein. Vorzugsweise ist er geradkettig und hat 2 bis 8 C-Atome. Er bedeutet demnach besonders Vinyl, Prop-1- oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl oder Oct-1-, 2-, 3-, 4-, 5-, 6- oder Oct-7-enyl.

Im Falle der Bedeutung Alkenyloxy in den vorstehend oder nachfolgend angegebenen Gruppen oder Substituenten, insbesondere in R^{a}, kann der Alkenyloxyrest geradkettig oder verzweigt sein. Vorzugsweise ist er geradkettig und bedeutet demnach insbesondere Vinyloxy, Prop-1- oder Prop-2-enyloxy, But-1-, 2- oder But-3-enyloxy, Pent-1-, 2-, 3- oder Pent-4-enyloxy, Hex-1-, 2-, 3-, 4- oder Hex-5-enyloxy, Hept-1-, 2-, 3-, 4-, 5- oder Hept-6-enyloxy oder Oct-1-, 2-, 3-, 4-, 5-, 6- oder Oct-7-enyloxy.

Die folgenden Ausführungsbeispiele sollen die Erfindung erläutern, ohne sie zu begrenzen. Vor- und nachstehend bedeuten Prozentangaben Gewichtsprozent. Alle Temperaturen sind in Grad Celsius angegeben. Es wird die Abkürzung DBH für 1,3-Dibrom-5,5-dimethylhydanthoin verwendet.

### Ausführungsbeispiele

### 1. Beispiel

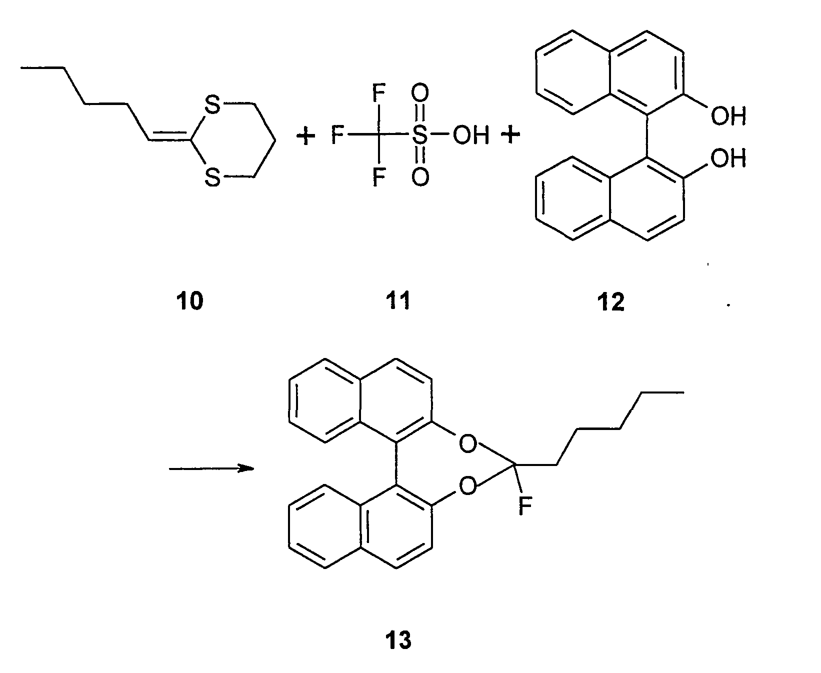

53,1 mmol des Thioacetals 10 werden in 70 ml Dichlormethan gelöst und unter Eiskühlung 53,1 mmol Trifluormethansulfonsäure 11 langsam zugegeben und noch 30 min gerührt. Anschließend wird der Ansatz auf -70 °C abgekühlt und innerhalb von 15 min eine Lösung von 64,9 mmol Triethylamin und 23,9 mmol (-)-S-1,1'-Bi-(2-naphthol) 12 in 50 ml Dichlormethan zugetropft. Nach weiteren 45 min werden langsam 265,5 mmol Triethylamin-trishydrofluorid hinzugegeben und dann innerhalb von 60 min portionsweise mit einer Suspension von 265,5 mmol DBH in 120 ml Dichlormethan versetzt. Man lässt noch 90 min rühren, den Ansatz auf -20 °C erwärmen und gibt die orangefarbene Suspension vorsichtig auf eine eiskalte Mischung aus 1 I ca. 1 M Natronlauge und 100 ml Natriumhydrogensulfitlösung. Die wäßrige Phase wird abgetrennt, dreimal mit Dichlormethan extrahiert und die vereinigten organischen Phasen werden wie üblich aufgearbeitet. Das Produkt weist einen Schmelzpunkt von etwa 138°C auf. Die massenspektrometrische und NMR-Analyse ergeben die erwarteten Signale.

### 2. Beispiel

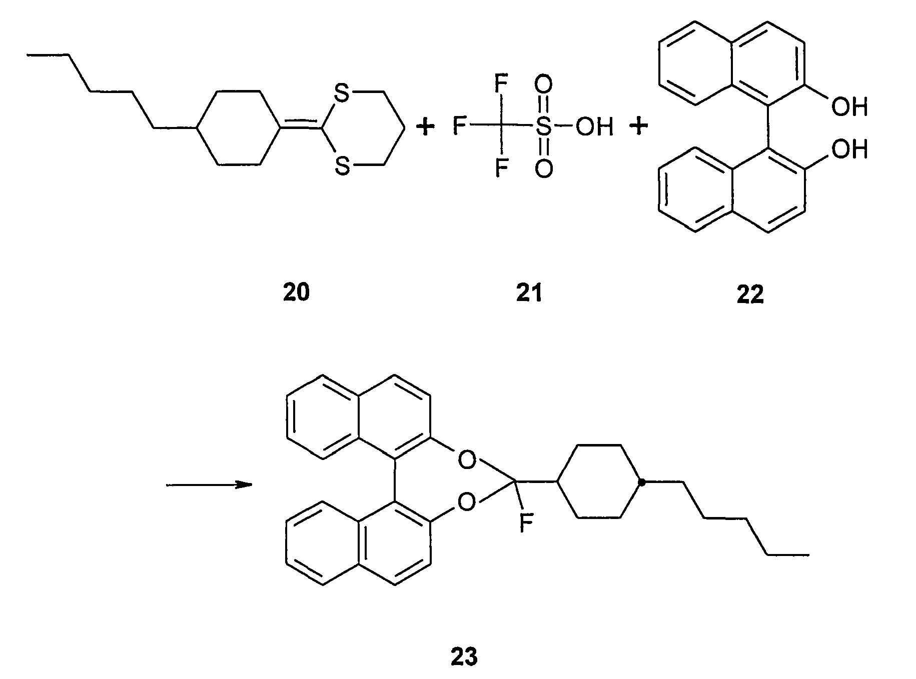

37,0 mmol des Thioacetals 20 werden in 50 ml Dichlormethan gelöst und unter Eiskühlung 37,0 mmol Trifluormethansulfonsäure 21 langsam zugeben. Nach 40 min wird die Kühlung entfernt und die gelbe Lösung noch 50 min bei 20 °C gerührt. Anschließend wird der Ansatz auf -70 °C abgekühlt und eine Lösung von 37,0 mmol Triethylamin und 16,6 mmol (-)-S-1,1'-Bi-(2-naphthol) 22 in 30 ml Dichlormethan zugetropft. Nach 90 min gibt man langsam 155 mmol Triethylamin-trishydrofluorid hinzu und versetzt dann innerhalb von 45 min portionsweise mit einer Suspension von 184,8 mmol DBH in 80 ml Dichlormethan. Nach 60 min läßt man den Ansatz auf -20 °C erwärmen und gibt die orangefarbene Suspension vorsichtig auf eine eiskalte Mischung aus 1 I ca. 1 M Natronlauge und 100 ml Natriumhydrogensulfitlösung. Die wäßrige Phase wird abgetrennt, dreimal mit Pentan extrahiert und die vereinigten organischen Phasen werden wie üblich aufgearbeitet. Das Produkt weist einen Schmelzpunkt von etwa 143 °C auf. Die massenspektrometrische und NMR-Analyse ergeben die erwarteten Signale.

### 3. Beispiel

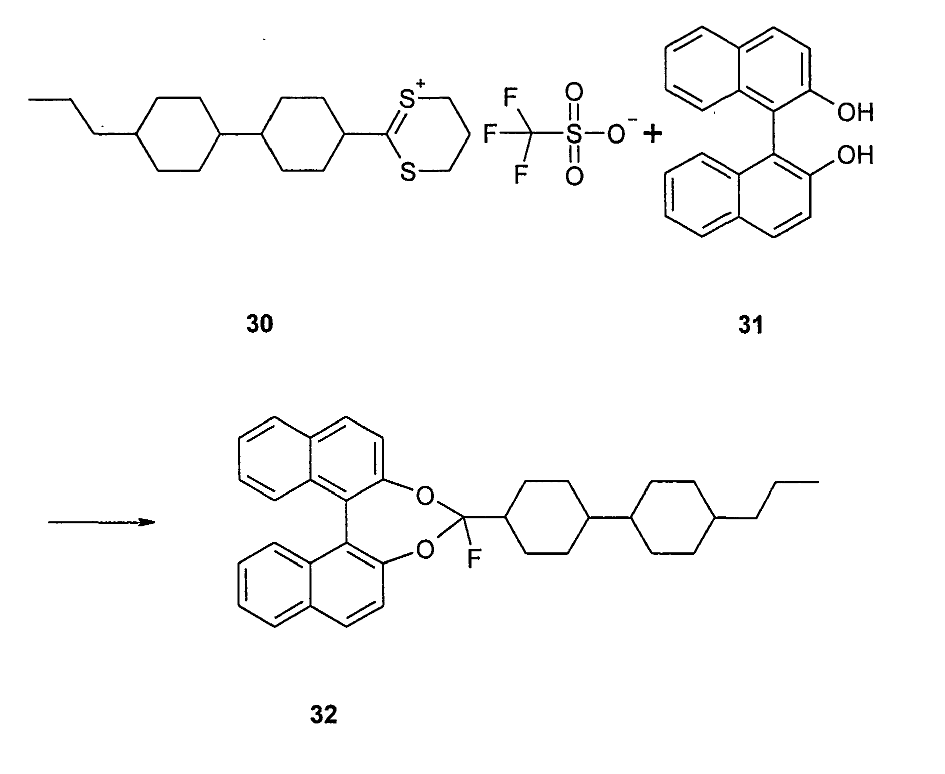

76,8 mmol des Dithianyliumtriflats 30 werden in 300 ml Dichlormethan vorgelegt und bei -65 °C eine Lösung von 34,9 mmol R(+)-1,1'-Bi-(2-naphthol) 31 und 38,4 mmol Triethylamin in 50 ml Dichlormethan zutropfen lassen. Man läßt noch 90 min rühren, gibt langsam Triethylamin-trishydrofluorid (0,38 mol) hinzu und versetzt dann innerhalb von 40 min portionsweise mit einer Suspension von 0,38 mol DBH in 150 ml Dichlormethan. Nach 60 min läßt man den Ansatz auf -20 °C erwärmen und gibt die orangefarbene Suspension vorsichtig auf eine eiskalte Mischung aus 1,5 I 1 M Natronlauge und 150 ml Natriumhydrogensulfitlösung. Die wäßrige Phase wird abgetrennt, dreimal mit Dichlormethan extrahiert und die vereinigten organischen Phasen werden wie üblich aufgearbeitet. Die massenspektrometrische und NMR-Analyse ergeben die erwarteten Signale.

### 4. Beispiel

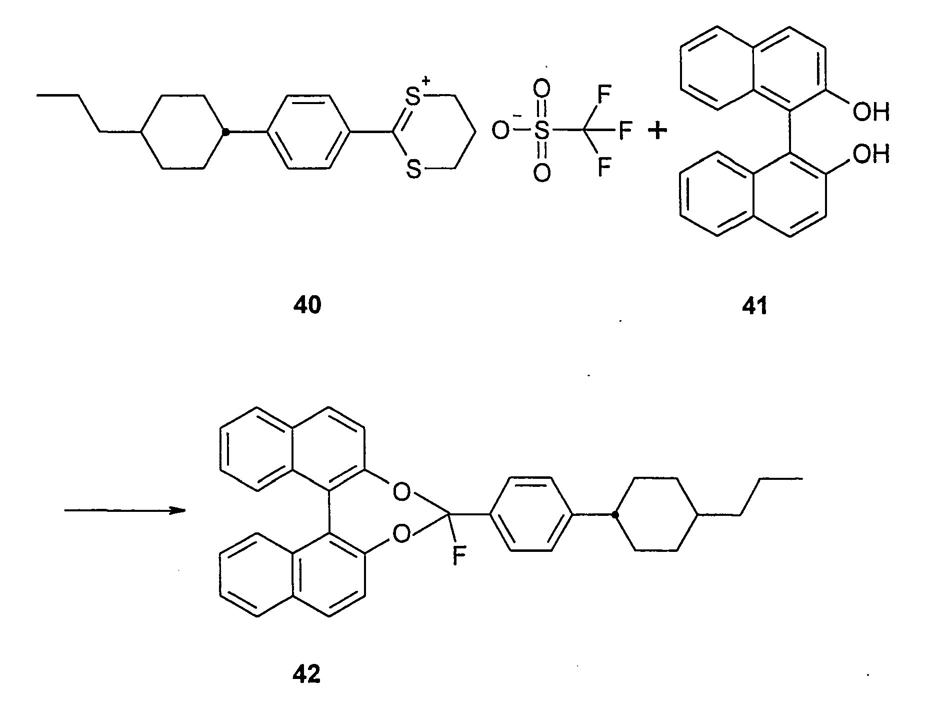

34,9 mmol S(-)-1,1'-Bi-(2-naphthol) 41 werden in 120 ml Dichlormethan vorgelegt, 76,8 mmol Triethylamin hinzugefügt und bei -65 °C innerhalb 1 h eine Lösung von 34,9 mmol des Dithianyliumtriflats 40 in 80 ml Dichlormethan zugetropft. Man läßt noch 1 h rühren, gibt langsam 0,15 mol Triethylamin-trishydrofluorid hinzu und versetzt dann innerhalb von 90 min portionsweise mit einer Suspension von 0,105 mol DBH in 50 ml Dichlormethan.

Nach 60 min läßt man den Ansatz auf -20 °C erwärmen und gibt die orangefarbene Suspension vorsichtig auf eine eiskalte Mischung aus 500 ml 1 M Natronlauge und 50 ml Natriumhydrogensulfitlösung (pH = 7). Die wäßrige Phase wird abgetrennt, dreimal mit Dichlormethan extrahiert, die vereinigten organischen Phasen werden wie üblich aufgearbeitet. Das Produkt weist einen Schmelzpunkt von etwa 228 °C auf. Die massenspektrometrische und NMR-Analyse ergeben die erwarteten Signale.

Analog zu den vorherigen Ausführungsbeispielen werden folgende Verbindungen, definiert durch die Gruppe W und den Rest R¹ gemäß Formel I, erhalten:

| **Nr.** | **W** | **R**¹ |
|---|---|---|
| 50 | 1,1'-Binaphthalin-2,2'-diyl | -C₃H₇ |
| 51 | 1,1'-Binaphthalin-2,2'-diyl | -C₄H₉ |
| 52 | 1,1'-Binaphthalin-2,2'-diyl | -C₆H₁₃ |
| 53 | 1,1'-Binaphthalin-2,2'-diyl | 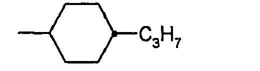 |
| 54 | 1,1'-Binaphthalin-2,2'-diyl | 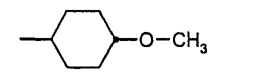 |
| 55 | 1,1'-Binaphthalin-2,2'-diyl | 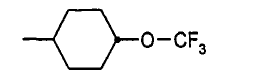 |
| 56 | 1,1'-Binaphthalin-2,2'-diyl | 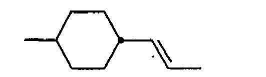 |
| 57 | 1,1'-Binaphthalin-2,2'-diyl | 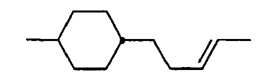 |
| 58 | 1,1'-Binaphthalin-2,2'-diyl | 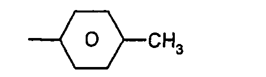 |
| 59 | 1,1'-Binaphthalin-2,2'-diyl | 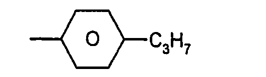 |
| 60 | 1,1'-Binaphthalin-2,2'-diyl | 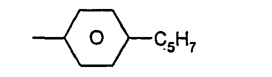 |
| 61 | 1,1'-Binaphthalin-2,2'-diyl | 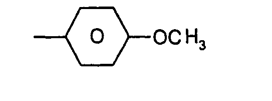 |
| 62 | 1,1'-Binaphthalin-2,2'-diyl | 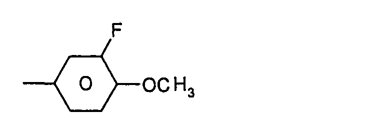 |
| 63 | 1,1'-Binaphthalin-2,2'-diyl | 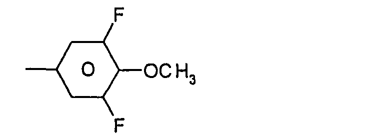 |
| 64 | 1,1'-Binaphthalin-2,2'-diyl | 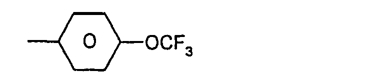 |
| 65 | 1,1'-Binaphthalin-2,2'-diyl | 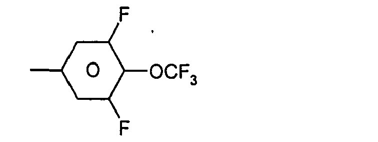 |
| 66 | 1,1'-Binaphthalin-2,2'-diyl | 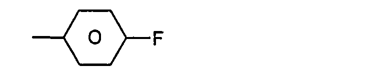 |
| 67 | 1,1'-Binaphthalin-2,2'-diyl | 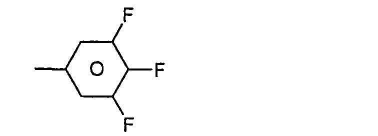 |
| 68 | 1,1'-Binaphthalin-2,2'-diyl | 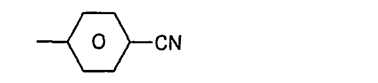 |
| 69 | 1,1'-Binaphthalin-2,2'-diyl | 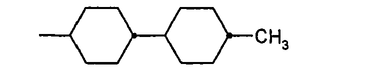 |
| 70 | 1,1'-Binaphthalin-2,2'-diyl | 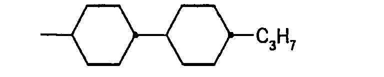 |
| 71 | 1,1'-Binaphthalin-2,2'-diyl | 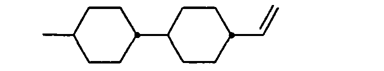 |
| 72 | 1,1'-Binaphthalin-2,2'-diyl | 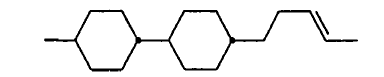 |
| 73 | 1,1'-Binaphthalin-2,2'-diyl | 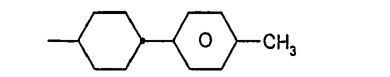 |
| 74 | 1,1'-Binaphthalin-2,2'-diyl | 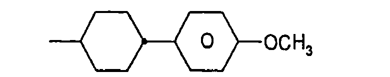 |
| 75 | 1,1'-Binaphthalin-2,2'-diyl | 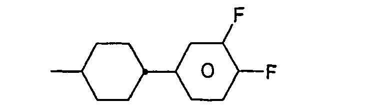 |
| 76 | 1,1'-Binaphthalin-2,2'-diyl | 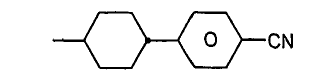 |
| 77 | 1,1'-Binaphthalin-2,2'-diyl | 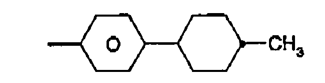 |
| 78 | 1,1'-Binaphthalin-2,2'-diyl | 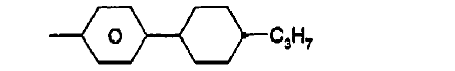 |
| 79 | 1,1'-Binaphthalin-2,2'-diyl | 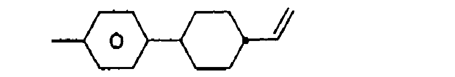 |
| 80 | 1,1'-Binaphthalin-2,2'-diyl | 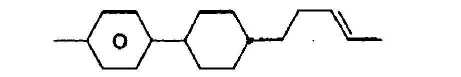 |
| 81 | 1,1'-Binaphthalin-2,2'-diyl | 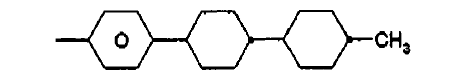 |
| 82 | 1,1'-Binaphthalin-2,2'-diyl | 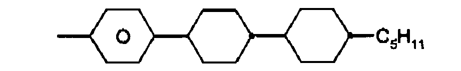 |
| 83 | 1,1'-Binaphthalin-2,2-diyl | 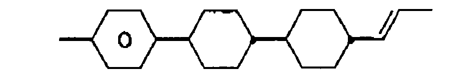 |
| 84 | 1,1'-Binaphthalin-2,2'-diyl | 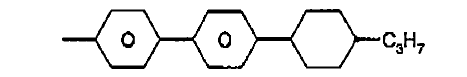 |
| 85 | 1,1'-Binaphthalin-2,2'-diyl | 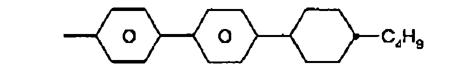 |
| 86 | 1,1'-Biphenyl-2,2'-diyl | -C₅H₁₁ |
| 87 | 1,1'-Biphenyl-2,2'-diyl | 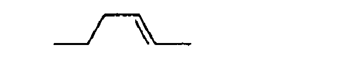 |
| 88 | 1,1'-Biphenyl-2,2'-diyl | 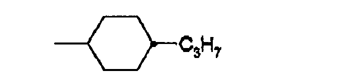 |
| 89 | 1,1'-Biphenyl-2,2'-diyl | 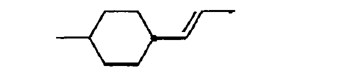 |
| 90 | 1,1'-Biphenyl-2,2'-diyl | 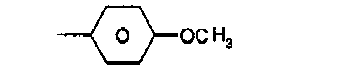 |
| 91 | 1,1'-Biphenyl-2,2'-diyl | 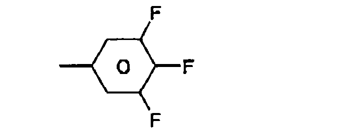 |
| 92 | 1,1'-Biphenyl-2,2'-diyl | 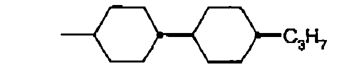 |
| 93 | 1,1'-Biphenyl-2,2'-diyl | 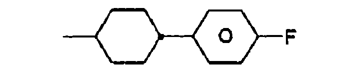 |
| 94 | 1,1'-Biphenyl-2,2'-diyl | 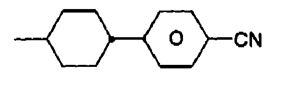 |
| 95 | 1,1'-Biphenyl-2,2'-diyl | 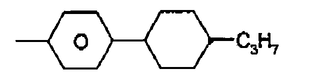 |
| 96 | 1,1'-Biphenyl-2,2'-diyl | 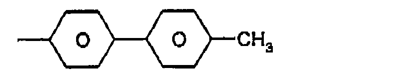 |
| 97 | 1,1'-Biphenyl-2,2'-diyl | 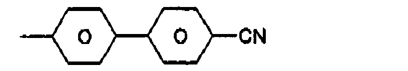 |
| 98 | 1,1'-Biphenyl-2,2'-diyl | 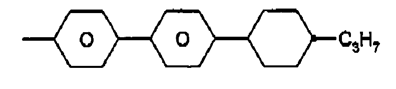 |

## Patentansprüche

1. Verfahren zur Herstellung von cyclischen Carbonsäureorthoesterfluoriden, bei dem
a) mindestens ein Bis(alkylthio)carbeniumsalz der Formel II 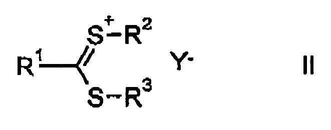 worin
R¹ geradkettiges, verzweigtes oder cyclisches Alkyl mit 1 bis 25 C-Atomen, worin ein oder mehrere H-Atome durch Halogen, -CN, weitere gegebenenfalls substituierte Alkyl- und/ oder Arylreste ersetzt sein können, und/ oder worin ein oder mehrere nicht benachbarte -CH₂-Gruppen unabhängig voneinander durch -CO-, -O-CO-, -CO-O-, -O-, -S-, -CH=CH-, -C≡C-, -NH- oder -N(CH₃)- ersetzt sein können, und/ oder Aryl, das ein oder mehrfach mit Halogen, geradkettigem, verzweigtem und/ oder cyclischem Alkyl und/ oder Aryl substituiert sein kann, worin auch ein oder mehrere CH-Gruppen durch N oder O ersetzt sein können, bedeutet,
R², R³ unabhängig voneinander geradkettiges, verzweigtes oder cyclisches Alkyl mit 1 bis 12 C-Atomen, wobei R², R³ derart
miteinander verbrückt sein können, dass die Gruppe 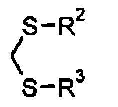 ein 4- bis 8-gliedriger Ring ist, und/oder worin ein oder mehrere H-Atome durch Halogen, weitere gegebenenfalls substituierte Alkyl- und/ oder Arylreste ersetzt sein können, und/ oder worin ein oder mehrere nicht benachbarte -CH₂-Gruppen unabhängig voneinander durch -CO-, -O-, -S-, -CH=CH-, -C≡C-, -NH- oder -N(CH₃)- ersetzt sein können, und/ oder Aryl, das ein oder mehrfach mit Halogen, geradkettigem, verzweigtem und/ oder cyclischem Alkyl und/ oder Aryl substituiert sein kann, bedeuten und
Y⁻ ein nicht- oder schwach-koordinierendes Anion ist, in Gegenwart mindestens einer Base mit mindestens einer organischen, mindestens zwei Hydroxylgruppen aufweisenden Verbindung der Formel III 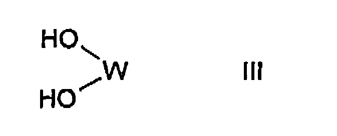
worin
W 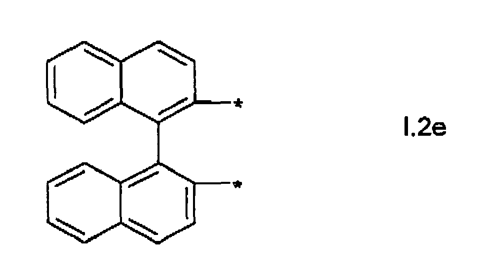 umgesetzt wird
b) und anschließend, vorzugsweise in situ, der erhaltene Thioorthoester mit einem Fluorierungsmittel und einem Oxidationsmittel zum cyclischen Carbonsäureorthoesterfluorid der Formel I 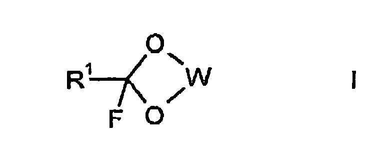 worin R¹ und W die angegebenen Bedeutungen besitzen, oxidativ fluorodesulfuriert wird.

2. Verfahren nach Anspruch 1, **dadurch gekennzeichnet, dass** als organische, mindestens zwei Hydroxylgruppen aufweisende Verbindung ein Alkandiol, eine aromatische oder heteroaromatische Dihydroxyverbindung oder ein Hydroxyalkylphenol, die durch ein oder mehrere Halogenatome und/ oder Alkylgruppen substituiert sein können, umgesetzt wird.

3. Verfahren nach Anspruch 1 oder 2, **dadurch gekennzeichnet, dass** Y ein Halogenid, Tetrafluoroborat, Hexafluorophosphat, Perchlorat oder Alkyl- oder Arylcarboxylat oder Alkyl- oder Arylsulfonat-Anion ist, wobei in den Alkyl- oder Arylgruppen ein, mehrere oder alle H-Atome durch Fluor oder Chlor substituiert sein können.

4. Verfahren nach einem der vorherigen Ansprüche, **dadurch gekennzeichnet, dass** das Oxidationsmittel eine Verbindung ist, die Haloniumäquivalente freisetzt, insbesondere ausgewählt ist aus der Gruppe, die gebildet ist von Dimethyldibromhydanthoin, N-Chlorsuccinimid, N-Bromsuccinimid, N-Jodsuccinimid, Dibromisocyanursäure, Chlor, Brom, SO₂Cl₂, SO₂ClF, Nitrosonium- und Nitroniumsalze, organische und anorganische Nitrite sowie Chloramin T.

5. Verfahren nach einem der vorherigen Ansprüche, **dadurch gekennzeichnet, dass** das Fluorierungsmittel ausgewählt ist aus der Gruppe, die gebildet ist von FluorwasserstofF, aliphatischen und aromatischen Amin-Fluorwasserstoff-Komplexen, insbesondere ausgewählt ist aus der Gruppe, die gebildet ist von Pyridin-, Triethylamin-, Melamin-, Polyvinylpyridin-Fluorwasserstoff-Komplexen.

6. Verfahren nach einem der vorherigen Ansprüche, **dadurch gekennzeichnet, dass** das Bis(alkylthio)carbeniumsalz zu der organischen, mindestens zwei Hydroxylgruppen aufweisenden Verbindung in einem Molverhältnis von kleiner gleich 2 : 1, insbesondere kleiner gleich 1,3 : 1 eingesetzt wird.

7. Verfahren nach einem der vorherigen Ansprüche, **dadurch gekennzeichnet, dass** das Bis(alkylthio)carbeniumsalz durch Addition einer Säure an ein Ketendithioketal erhalten wird

8. Verfahren nach Anspruch 7, **dadurch gekennzeichnet, dass** das Bis(alkykhio)carbeniumsalz der Formel II durch Addition einer Säure HY, worin Y die in Anspruch 1 oder 3 angegebene Bedeutung hat, an ein Ketendithioketal der Formel IV 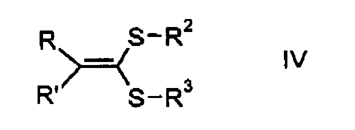 worin R² und R³ die in Anspruch 1 angegebene Bedeutung besitzen und
R, R' unabhängig voneinander derart eine der in Anspruch 1 für R¹ angegebenen Bedeutungen, einschließlich H, besitzen, dass die Gruppe 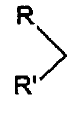 die selbe Bedeutung wie R¹ besitzt,
erhalten wird.

9. Verfahren nach Anspruch 7 oder 8, **dadurch gekennzeichnet, dass** das Ketendithioketal aus einer Carbonylverbindung durch Umsetzung mit einer oder mehreren Thiolverbindungen erhalten wird.

10. Cyclische Carbonsäureorthoesterfluoride der Formel I 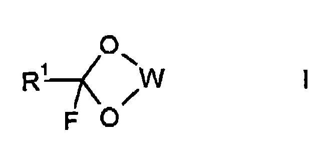 worin W und R¹ die in Anspruch 1 angegebenen Bedeutungen besitzen.

11. Cyclische Carbonsäureorthoesterfluoride der Formel I laut Anspruch 10, worin R¹ einen Rest der Formel 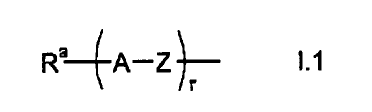 bedeutet, in der
R^{a} H, Halogen, -CN, -NCS, -SF₅ oder Alkyl mit 1 bis 12 C-Atomen, worin auch eine oder zwei nicht benachbarte -CH₂-Gruppen durch -O-, -S-, -CO-, -O-CO-, -CO-O-, -E- und/ oder -C≡C- ersetzt sein können und/ oder worin auch eine oder mehrere H-Atome durch Halogen und/ oder -CN ersetzt sein können, bedeutet,
E CR⁴=CR⁵ oder CHR⁴-CHR⁵,
R⁴, R⁵ jeweils unabhängig voneinander H, Alkyl mit 1-6 C-Atomen, F, Cl, Br, CF₃ oder CN bedeutet,
Z -O-CO-, -CO-O-, -C₂H₄-, -CH₂-CF₂-, -CF₂-CH₂-, -CF₂-CF₂-, -(CF₂)₃-, -(CF₂)₄-, -CH₂-O-, -CF₂-O-, -O-CH₂-, -O-CF₂-, -CH=CH-, -CF=CH-, -CH=CF-, -CF=CF-, -C≡C- oder eine Einfachbindung,
A 1,4-Phenylen, worin ein oder mehrere CH-Gruppen durch N ersetzt sein können, 1,4-Cyclohexylen, worin ein oder zwei nicht benachbarte CH₂-Gruppen durch O und/ oder S ersetzt sein können, 1,4-Cyclohexenylen, 1,4-Bicyclo(2,2,2)octylen, Piperidin-1,4-diyl, Naphthalin-2,6-diyl, Decahydronaphthalin-2,6-diyl oder 1,2,3,4-Tetrahydronaphthalin-2,6-diyl, wobei in diesen Gruppen ein oder mehrere H-Atome substituiert sein können durch Halogen, -CN und/ oder gegebenenfalls ein- oder mehrfach halogeniertes Alkyl mit 1 bis 6 C-Atomen, und
r 1, 2 oder 3 ist, wobei mehrfach vorkommende Gruppen A und/ oder Z gleiche oder verschiedene Bedeutungen besitzen können.

## Claims

1. Process for the preparation of cyclic carboxylic acid orthoester fluorides in which
a) at least one bis(alkylthio)carbenium salt of the formula II 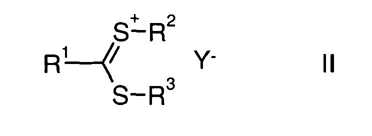 in which
R¹ denotes straight-chain, branched or cyclic alkyl having 1 to 25 C atoms, in which one or more H atoms may be replaced by halogen, -CN, further optionally substituted alkyl and/or aryl radicals, and/or in which one or more non-adjacent -CH₂- groups may be replaced, independently of one another, by -CO-, -O-CO-, -CO-O-, -O-, -S-, -CH=CH-, -C≡C-, -NH- or -N(CH₃)-, and/or aryl, which may be mono- or polysubstituted by halogen, straight-chain, branched and/or cyclic alkyl and/or aryl, and in which one or more CH groups may be replaced by N or O,
R², R³, independently of one another, denote straight-chain, branched or cyclic alkyl having 1 to 12 C atoms, where R², R³ may be bridged to one another in such a way that the group 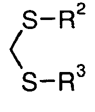 is a 4- to 8-membered ring, and/or in which one or more H atoms may be replaced by halogen, further optionally substituted alkyl and/or aryl radicals, and/or in which one or more non-adjacent -CH₂- groups may be replaced, independently of one another, by -CO-, -O-, -S-, -CH=CH-, -C≡C-, -NH- or -N(CH₃)-, and/or aryl, which may be mono- or polysubstituted by halogen, straight-chain, branched and/or cyclic alkyl and/or aryl, and
Y⁻ is a non- or weakly coordinating anion,
is reacted with at least one organic compound containing at least two hydroxyl groups of the formula III 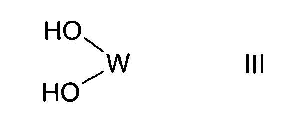 in which
W denotes 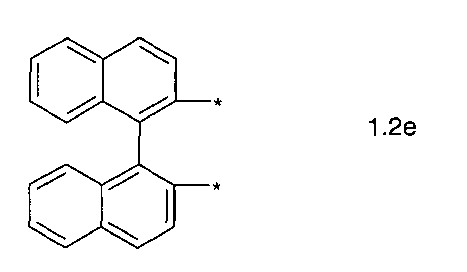
in the presence of at least one base,
b) and subsequently, preferably in situ, the resultant thioorthoester is subjected to oxidative fluorodesulfurisation using a fluorinating agent and an oxidant to give the cyclic carboxylic acid orthoester fluoride of the formula I 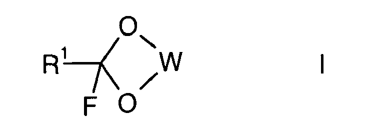
in which R¹ and W have the meanings indicated.

2. Process according to Claim 1, **characterised in that** the organic compound containing at least two hydroxyl groups that is reacted is an alkanediol, an aromatic or heteroaromatic dihydroxyl compound or an hydroxyalkylphenol, each of which may be substituted by one or more halogen atoms and/or alkyl groups.

3. Process according to Claim 1 or 2, **characterised in that** Y⁻ is a halide, tetrafluoroborate, hexafluorophosphate, perchlorate or alkyl- or arylcarboxylate or alkyl- or arylsulfonate anion, where one, a number or all of the H atoms in the alkyl or aryl groups may be substituted by fluorine or chlorine.

4. Process according to one of the preceding claims, **characterised in that** the oxidant is a compound which liberates halonium equivalents, in particular selected from the group formed by dimethyldibromohydantoin, N-chlorosuccinimide, N-bromosuccinimide, N-iodosuccinimide, dibromoisocyanuric acid, chlorine, bromine, SO₂Cl₂, SO₂CIF, nitrosonium and nitronium salts, organic and inorganic nitrites and chloramine-T.

5. Process according to one of the preceding claims, **characterised in that** the fluorinating agent is selected from the group formed by hydrogen fluoride, aliphatic and aromatic amine/hydrogen fluoride complexes, in particular selected from the group formed by pyridine, triethylamine, melamine, polyvinylpyridine/hydrogen fluoride complexes.

6. Process according to one of the preceding claims, **characterised in that** the bis(alkylthio)carbenium salt is employed in a molar ratio of less than or equal to 2 : 1, in particular less than or equal to 1.3 : 1, to the organic compound containing at least two hydroxyl groups.

7. Process according to one of the preceding claims, **characterised in that** the bis(alkylthio)carbenium salt is obtained by addition of an acid onto a ketene dithioketal.

8. Process according to Claim 7, **characterised in that** the bis(alkylthio)-carbenium salt of the formula II is obtained by addition of an acid HY, in which Y has the meaning indicated in Claim 1 or 3, onto a ketene dithioketal of the formula IV 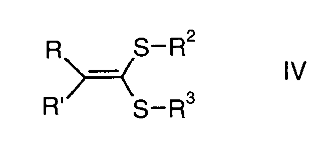 in which R² and R³ have the meaning indicated in Claim 1, and
R, R', independently of one another, have one of the meanings indicated for R¹ in Claim 1, including H, in such a way that the group 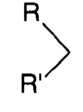 has the same meaning as R¹.

9. Process according to Claim 7 or 8, **characterised in that** the ketene dithioketal is obtained from a carbonyl compound by reaction with one or more thiol compounds.

10. Cyclic carboxylic acid orthoester fluorides of the formula I 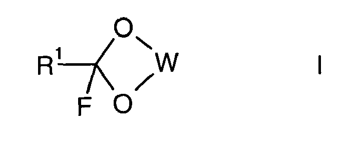 in which W and R¹ have the meanings indicated in Claim 1.

11. Cyclic carboxylic acid orthoester fluorides of the formula I according to Claim 10, in which R¹ denotes a radical of the formula 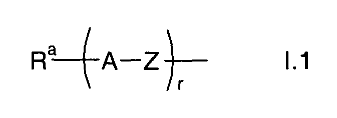 in which
R^{a} denotes H, halogen, -CN, -NCS, -SF₅ or alkyl having 1 to 12 C atoms, in which, in addition, one or two non-adjacent -CH₂-groups may be replaced by -O-, -S-, -CO-, -O-CO-, -CO-O-, -E- and/or -C≡C- and/or in which, in addition, one or more H atoms may be replaced by halogen and/or -CN,
E denotes CR⁴=CR⁵ or CHR⁴-CHR⁵,
R⁴, R⁵ each, independently of one another, denote H, alkyl having 1-6 C atoms, F, Cl, Br, CF₃ or CN,
Z denotes -O-CO-, -CO-O-, -C₂H₄-, -CH₂-CF₂-, -CF₂-CH₂-, -CF₂-CF₂-, -(CF₂)₃-, -(CF₂)₄-, -CH₂-O-, -CF₂-O-, -O-CH₂-, -O-CF₂-, -CH=CH-, -CF=CH-, -CH=CF-, -CF=CF-, -C=C- or a single bond,
A denotes 1,4-phenylene, in which one or more CH groups may be replaced by N, 1,4-cyclohexylene, in which one or two non-adjacent CH₂ groups may be replaced by O and/or S, 1,4-cyclohexenylene, 1,4-bicyclo[2.2.2]octylene, piperidine-1,4-diyl, naphthalene-2,6-diyl, decahydronaphthalene-2,6-diyl or 1,2,3,4-tetrahydronaphthalene-2,6-diyl, where one or more H atoms in these groups may be substituted by halogen, -CN and/or optionally mono- or polyhalogenated alkyl having 1 to 6 C atoms, and
r is 1, 2 or 3, where groups A and/or Z which occur more than once may have identical or different meanings.

## Revendications

1. Procédé de préparation de fluorures d'orthoester d'acide carboxylique cyclique dans lequel
a) au moins un sel de bis(alkylthio)carbénium de formule II 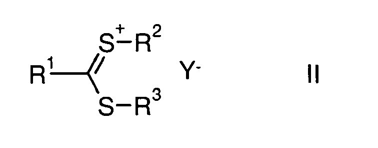 dans laquelle
R¹ désigne alkyle à chaîne linéaire, ramifiée ou cyclique ayant de 1 à 25 atomes de C, dans lequel un ou plusieurs atomes de H peuvent être remplacés par halogène, -CN, d'autres radicaux alkyle et/ou aryle éventuellement substitués, et/ou dans lequel un ou plusieurs groupements -CH₂-non adjacents peuvent être remplacés, indépendamment les uns des autres, par -CO-, -O-CO-, -CO-O-, -O-, -S-, -CH=CH-, -C≡C-, -NH- ou -N(CH₃)-, et/ou aryle, pouvant être mono- ou polysubstitué par halogène, alkyle à chaîne linéaire, ramifiée et/ou cyclique et/ou aryle, et dans lequel un ou plusieurs groupements CH peuvent être remplacés par N ou O,
R², R³, indépendamment l'un de l'autre, désignent alkyle à chaîne linéaire, ramifiée ou cyclique ayant de 1 à 12 atomes de C, où R², R³ peuvent être pontés l'un à l'autre de telle sorte que le groupement 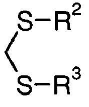 soit un cycle de 4 à 8 chaînons, et/ou dans lequel un ou plusieurs atomes de H peuvent être remplacés par halogène, d'autres radicaux alkyle et/ou aryle éventuellement substitués, et/ou dans lequel un ou plusieurs groupements -CH₂- non adjacents peuvent être remplacés, indépendamment les uns des autres, par-CO-, -O-, -S-, -CH=CH-, -C≡C-, -NH- ou -N(CH₃)-, et/ou aryle, pouvant être mono- ou polysubstitué par halogène, alkyle à chaîne linéaire, ramifiée et/ou cyclique et/ou aryle, et
Y⁻ est un anion de coordination faible ou nulle,
est réagi avec au moins un composé organique contenant au moins deux groupements hydroxyle de formule III 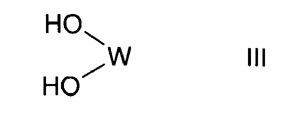 dans laquelle
W désigne 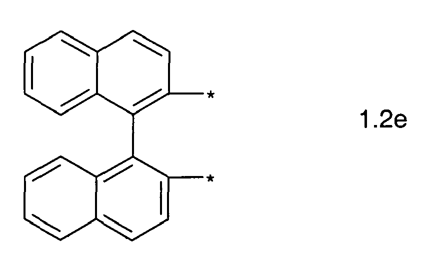
en présence d'au moins une base,
b) et ensuite, préférablement in situ, le thioorthoester résultant est soumis à une fluorodésulfurisation oxydative à l'aide d'un agent de fluoration et d'un oxydant pour donner le fluorure d'orthoester d'acide carboxylique cyclique de formule I 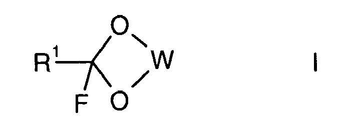
dans laquelle R¹ et W ont les significations indiquées.

2. Procédé selon la revendication 1, **caractérisé en ce que** le composé organique contenant au moins deux groupements hydroxyle étant réagi est un alcanediol, un composé dihydroxylé aromatique ou hétéroaromatique ou un hydroxyalkylphénol, chacun d'entre eux pouvant être substitué par un ou plusieurs atomes d'halogène et/ou des groupements alkyle.

3. Procédé selon la revendication 1 ou 2, **caractérisé en ce que** Y⁻ est un anion halogénure, tétrafluoroborate, hexafluorophosphate, perchlorate ou alkyl- ou arylcarboxylate ou alkyl- ou arylsulfonate, où l'un, un certain nombre ou l'ensemble des atomes de H dans les groupements alkyle ou aryle peut être substitué par fluor ou chlore.

4. Procédé selon l'une des revendications précédentes, **caractérisé en ce que** l'oxydant est un composé libérant des équivalents halogénium, choisi en particulier parmi le groupe constitué par la diméthyldibromohydantoïne, le N-chlorosuccinimide, le N-bromosuccinimide, le N-iodosuccinimide, l'acide dibromoisocyanurique, le chlore, le brome, SO₂Cl₂, SO₂CIF, les sels de nitrosonium et de nitronium, les nitrites organiques et inorganiques et la chloramine-T.

5. Procédé selon l'une des revendications précédentes, **caractérisé en ce que** l'agent de fluoration est choisi parmi le groupe constitué par le fluorure d'hydrogène, les complexes d'amine aliphatique et aromatique/fluorure d'hydrogène, en particulier choisi parmi le groupe constitué par les complexes de pyridine, triéthylamine, mélamine, polyvinylpyridine/fluorure d'hydrogène.

6. Procédé selon l'une des revendications précédentes, **caractérisé en ce que** le sel de bis(alkylthio)carbénium est employé selon un rapport molaire inférieur ou égal à 2:1, en particulier inférieur ou égal à 1,3:1, par rapport au composé organique contenant au moins deux groupements hydroxyle.

7. Procédé selon l'une des revendications précédentes, **caractérisé en ce que** le sel de bis(alkylthio)carbénium est obtenu par l'addition d'un acide sur un dithioacétal de cétène.

8. Procédé selon la revendication 7, **caractérisé en ce que** le sel de bis(alkylthio)carbénium de formule II est obtenu par l'addition d'un acide HY, dans lequel Y a la signification indiquée selon la revendication 1 ou 3, sur un dithioacétal de cétène de formule IV 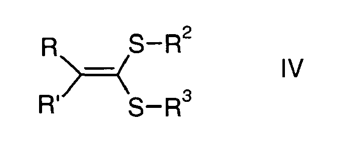 dans laquelle R² et R³ ont la signification indiquée selon la revendication 1, et
R, R', indépendamment l'un de l'autre, ont l'une des significations indiquées pour R¹ selon la revendication 1, y compris H, de telle sorte que le groupement 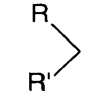 a la même signification que R¹.

9. Procédé selon la revendication 7 ou 8, **caractérisé en ce que** le dithioacétal de cétène est obtenu à partir d'un composé carbonyle par réaction avec un ou plusieurs composés thiol.

10. Fluorures d'orthoester d'acide carboxylique cyclique de formule I 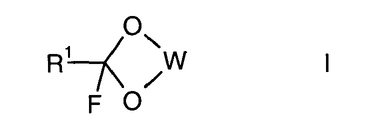 dans laquelle W et R¹ ont les significations indiquées selon la revendication 1.

11. Fluorures d'orthoester d'acide carboxylique cyclique de formule I selon la revendication 10, dans laquelle R' désigne un radical de formule 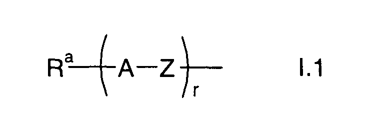 dans laquelle
R^{a} désigne H, halogène, -CN, -NCS, -SF₅ ou alkyle ayant de 1 à 12 atomes de C, dans laquelle, en outre, un ou deux groupements -CH₂- non adjacents peuvent être remplacés par -O-, -S-, -CO-, -O-CO-, -CO-O-, -E- et/ou -C≡C- et/ou dans laquelle, en outre, un ou plusieurs atomes de H peuvent être remplacés par halogène et/ou -CN,
E désigne CR⁴=CR⁵ ou CHR⁴-CHR⁵,
R⁴ R⁵ chacun, indépendamment l'un de l'autre, désignent H, alkyle ayant 1-6 atomes de C, F, CI, Br, CF₃ ou CN,
Z désigne -O-CO-, -CO-O-, -C₂H₄-, -CH₂-CF₂-, -CF₂-CH₂-, -CF₂-CF₂-, -(CF₂)₃-, -(CF₂)₄-, -CH₂-O-, -CF₂-O-, -O-CH₂-, -O-CF₂-, -CH=CH-, -CF=CH-, -CH=CF-, -CF=CF-, -C≡C- ou une liaison simple,
A désigne 1,4-phénylène, dans lequel un ou plusieurs groupements CH peuvent être remplacés par N, 1,4-cyclohexylène, dans lequel un ou deux groupements CH₂ non adjacents peuvent être remplacés par O et/ou S, 1,4-cyclohexenylène, 1,4-bicyclo[2.2.2]octylène, pipéridine-1,4-diyle, naphtalène2,6-diyle, décahydronaphtalène-2,6-diyle ou 1,2,3,4-tétrahydronaphtalène-2,6-diyle, où un ou plusieurs atomes de H dans ces groupements peuvent être substitués par halogène, -CN et/ou alkyle éventuellement mono- ou polyhalogéné ayant de 1 à 6 atomes de C, et
r vaut 1, 2 ou 3, où les groupements A et/ou Z présents plus d'une fois peuvent avoir des significations identiques ou différentes.
